# EUROPEAN PATENT APPLICATION

(11) **EP 3 650 015 A1**
(43) Date of publication of application: **13.05.2020**
(21) Application number: 18828227.1
(22) Date of filing: 14.02.2018
(51) Int. Cl.: A61K 9/20, A61K 9/48, A61K 9/14, A61K 9/68, A61P 31/18, A61P 31/12, A61P 31/20, C07D 411/04, C07C 311/46, C07F 9/6553, C07F 9/6568, C07F 9/6561

(54) **COMBINED MEDICINAL PREPARATION FOR TREATING VIRAL INFECTIONS**

(30) Priority: 03.07.2017 RU 2017123373
(71) Applicant: Ivachtchenko, Alexandre Vasilievich, Hallandale Beach, FL 33009 (US); Ivashchenko, Andrey Alexandrovich, Moscow 127576 (RU); Savchuk, Nikolay Filippovich, Rancho Santa Fe, CA 92067 (US); Alla Chem, LLC, Hallandale Beach, FL 33009 (US); Ivachtchenko, Alena Alexandrovna, Hallandale, Florida 33009 (US)
(72) Inventor: IVACHTCHENKO, Alexandre Vasilievich, Florida, Hallandale Beach 33009 (US); IVASHCHENKO, Andrey Alexandrovich, Florida, Hallandale Beach 33009 (US); SAVCHUK, Nikolay Filippovich, Florida, Hallandale Beach 33009 (US)
(74) Representative: Zellentin & Partner mbB Patentanwälte
(86) International application number: PCT/RU2018/000087
(87) International publication number: WO 2019/009759

(57) **Abstract**

The present invention relates to a novel combination drug in a solid oral dosage form comprising, as one of the three active ingredients, elsulfavirine sodium that may be suitable for medical use when treating viral infections including HIV and HBV.

An antiviral combination drug in a solid oral dosage form comprising, as one of the three active ingredients, a therapeutically effective amount of elsulfavirine sodium of formula **1a** in a crystalline or polycrystalline form optionally in combination with auxiliary agents:

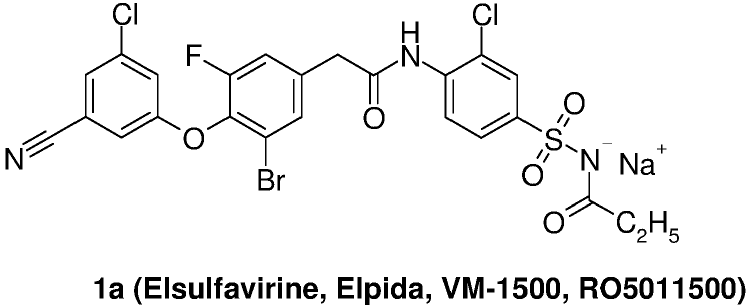

## Description

### Field of the invention

The present invention relates to a novel combination drug in a solid oral dosage form comprising, as one of the three active ingredients, elsulfavirine sodium to be used in medicine for treating viral infections including HIV and hepatitis B virus (HBV).

### Background of the invention

The human immunodeficiency virus (HIV) is a lentivirus (a subgroup of retroviruses) that causes an indolent disease - HIV-infection [Weiss R.A. How does HIV cause AIDS. Science 1993, 260 (5112), 1273-1279*.* Douek D.C., Roederer M., Koup R.A. Emerging Concepts in the Immunopathogenesis of AIDS». Annu. Rev. Med. 2009, 60, 471-84*].* The human immunodeficiency virus was independently discovered in 1983 at two laboratories: one by a research team led by Luc Montagnier at the Pasteur Institute in France and the other, led by Robert Gallo at the National Cancer Institute in the United States. The findings discussing the first isolation of a new retrovirus from tissues of patients with symptoms of AIDS were published on May 20, 1983 in the journal *Science* [Barre-Sinoussi F. et al. Isolation of a T-lymphotropic retrovirus from a patient at risk for acquired immune deficiency syndrome (AIDS). Science 1983, 220 (4599), 868-871. Gallo R. C. at al. Isolation of human T-cell leukemia virus in acquired immune deficiency syndrome (AIDS). Science 1983, 220 (4599), 865-867.]. In 2008, Luc Montagnier and Françoise Barre-Sinoussi shared the Nobel Prize for Physiology and Medicine for their "discovery of the human immunodeficiency virus."

The HIV virus infects the cells of the immune system that have CD4 receptors on their surface: T helpers, monocytes, macrophages, Langerhans cells, dendritic cells, microglial cells. This leads to immunodepression and development of Acquired Immune Deficiency Syndrome (AIDS); loss of patients' body's ability to protect themselves against infections and tumors; emergence of secondary opportunistic diseases that are not typical for people with normal immune status. Without treatment, average survival time after infection with HIV is estimated to be 9 to 11 years, depending on the HIV subtype.

According to the worldwide statistics [http://www.lenoblspid.ru/news24/postid/own_news/1166], in 2015, 36.7 million people globally were living with HIV, 2.1 million people were newly infected with HIV, and 1.1 million people died of AIDS-related illnesses. Since the start of the epidemic, 78 million people have become infected with HIV, of which 35 million people have died of AIDS-related illnesses.

As of December 2015, 17 million people living with HIV were receiving antiretroviral therapy, while in June 2015 this number was 15.8 million people and in 2010, 7.5 million people. In 2015, 46% of all adults living with HIV had access to treatment, whereas in 2010, only 23%.

Since 2010, new HIV infections fell by 6%. In 2015, the global number of HIV-infected was 2.1 million people, while in 2010, 2.2 million people.

As compared to the highest figure in 2005, the rate of AIDS-associated mortality has declined by 45%, and in 2015, the number of individuals died due to AIDS worldwide was 1.1 million people against 2 million people in 2005.

HIV can be suppressed by combination antiretroviral therapy (ART) involving three or more antiretroviral drugs. ART does not cure HIV infection but suppresses viral replication within a person's body and allows the individual's immune system to strengthen and regain the capacity to fight off infections. Life expectancy for patients receiving ART may be extended to 70-80 years [http://www.who.int/mediacentre/factsheets/fs360/ru/].

Hepatitis B is an infectious inflammatory disease of the liver tissue resulting from the invasion of the human body by a hepatitis B virus (HBV). It is a major global health problem. It can cause chronic infection and puts people at high risk of death from cirrhosis and liver cancer.

According to the World health Organization (WHO), about 350 million people in the world have chronic hepatitis B. The highest prevalence is observed in Central and Southern Africa, a major part of Asia, Amazonia, the northern parts of Central and Eastern Europe, and Middle Eastern countries, where chronically infected individuals account for 5-10% of the adult population. High prevalence of chronic infections is also seen in the Amazon region and in the southern parts of Eastern and Central Europe. It is estimated that 2-5% of the population in the Middle East and Hindustan is chronically infected. In Western Europe and North America, chronically infected patients account for less than 1% of the population. Russia belongs to countries with medium hepatitis B prevalence (≈7% of population). By various estimates, the number of infected in the Russian Federation is as high as 3 to 6 mln people. The incidence of chronic forms of hepatitis B generally stands at 13-14 per 100 K people.

Hepatitis B virus belongs to the family of hepadnaviruses-hepatotropic DNA-contaning viruses. The concentration of hepatitis B virus in the blood during the progression of disease is extremely high and can reach up to 1012 viral particles per 1 ml of blood. The hepatitis B virus is very stable and survives in the ambient environment for one week. It is 100 times more infectious than HIV (human immunodeficiency virus). HBV causes an infectious disease-acute or chronic hepatitis B accompanied by a severe inflammatory liver injury. A major sign of this disease is the detection of HbsAg-the HBV surface antigen commonly referred to as Australian antigen-in the blood [http://58.rospotrebnadzor.ru/rss_all;jsessionid=1C1BE5CC6CC2130C60916F6C1 695C57C?p_auth=6WIU2BaT&p_p_id=101_INSTANCE_Kq6J&p_p_lifecycle=1&p _p_state=exclusive&p_p_mode=view&p_p_col_id=column1&p_p_col_count=1&_1 01_INSTANCE_Kq6J_struts_action=%2Fasset_publisher%2Fexport_journal_articl e&_101_INSTANCE_Kq6J_groupld=10156&_101_INSTANCE_Kq6J_articleld=17 9250&_101_INSTANCE_Kq6J_targetExtension=pdf].

The treatment of HBV infection in those in need thereof reduces the risk of hepatocellular carcinoma and death. It is estimated that therapy will be beneficial for 20-30% of HBV-infected individuals. However, anti-HBV drugs are not widely accessible or not used by HBV-infected [http://www.euro.who.int/en/health-topics/communicable-diseases/hepatitis/news/news/2011/11/treatment-of-chronic-hepatitis-b-virus-infection-in-resource-constrained-settings-expert-panel-consensus/russian-version-treatment-of-chronic-hepatitis-b-virus-infection-in-resource-constrained-settings-expert-panel-consensus].

HIV/HBV coinfection is a common occurrence. Chronic HBV infection occurs in 5-10% of HIV-infected individuals who acquire HBV 10 times more often than the general population [http://hivinsite.ucsf.edu/lnSite?page=kb-05-03-04#S1X].

Antiviral drugs currently recommended for treating infection induced by HIV fail to sufficiently suppress HBV replication; this becomes a topic of major concern regarding about 10% of HIV/HBV-coinfected in Africa. It has been found out that coinfected persons who do not take HBV suppressants acquire an advanced liver disease. In the light of these problems, WHO believes that chronic HBV infection is a serious public health problem in developing countries; all HIV-infected individuals must be screened for HBV; HIV/HBV-coninfected persons must receive ART that is effective against both viruses and reduces the probability of resistance development [http://www.euro.who.int/en/health-topics/ communicable-diseases/hepatitis/news/news/2011/11/ treatment-of-chronic-hepatitis-b-virus-infection-in-resource-constrained-settings-expert-panel-consensus/russian-version-treatment-of-chronic-hepatitis-b-virus-infection-in-resource-constrained-settings-expert-panel-consensus].

Examples of single-component drugs for ART can be elsulfavirine of formula 1 [WO2005/102989, RU 2389719, WO2010/028968], proinhibitors of formulas 2a-2I, lamivudine of formula 2a, and emtricitabine of formula 2b and derivatives thereof of formulas 2c-2I [https://www.accessdata.fda.gov/drugsatfda_docs/label/2013/021003s015,021004 s015lbl.pdf; https://www.accessdata.fda.gov/drugsatfda_docs/label/2012/021500s019lbl.pdf; US 152221613; RU 2017106609; RU 2017106610; RU 2017106611; RU 2017106615], tenofovir of formula 3 [https://www.accessdata.fda.gov/drugsatfda_docs/label/2012/022577lbl.pdf] and of formulas 4a-4k [WO 2013025788], [US 152221613], [RU 2017106609; RU 2017106610; RU 2017106611; RU 2017106615], rilpivirine hydrochloride of formula 5 [http://www.edurant.com/shared/prescribing-information-edurant.pdf], efavirenz of formula 6 [https://www.accessdata.fda.gov/drugsatfda_docs/label/2005/020972s026, 021360 s013lbl.pdf], elvitegravir of formula 7 [https://pubchem.ncbi.nlm.nih.gov/compound/Elvitegravir# section=2D-Structure] and cobicistat of formula 8 [https://pubchem.ncbi.nlm.nih.gov/compound/Cobicistat].

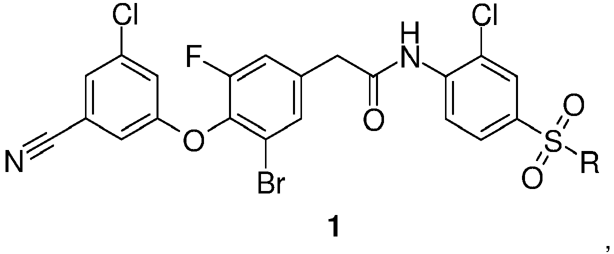

Wherein R is C₂H₅CON⁻Na⁺, NH₂;

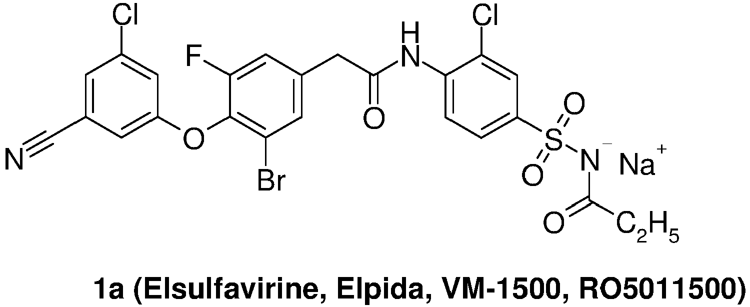

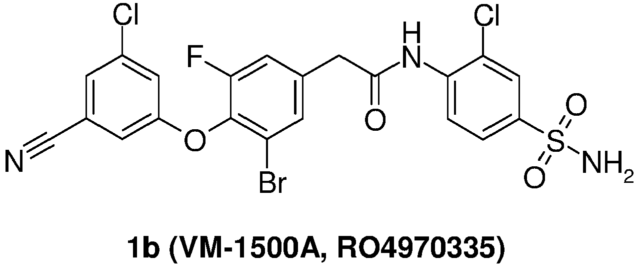

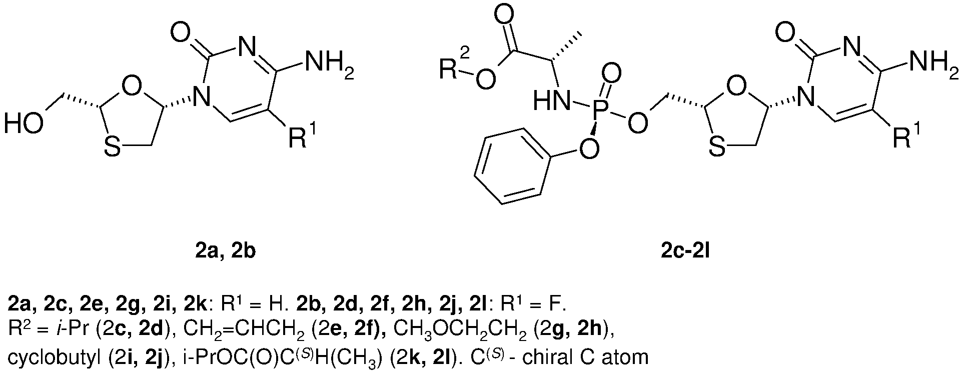

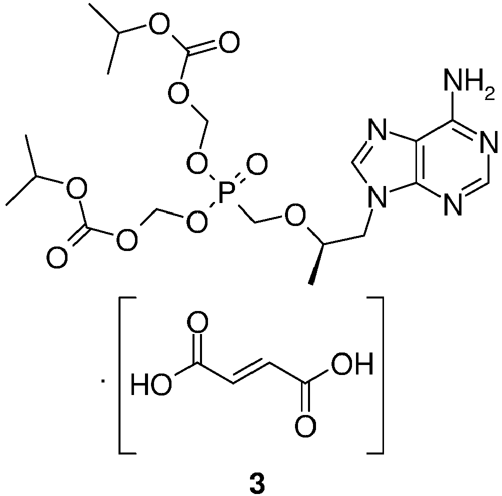

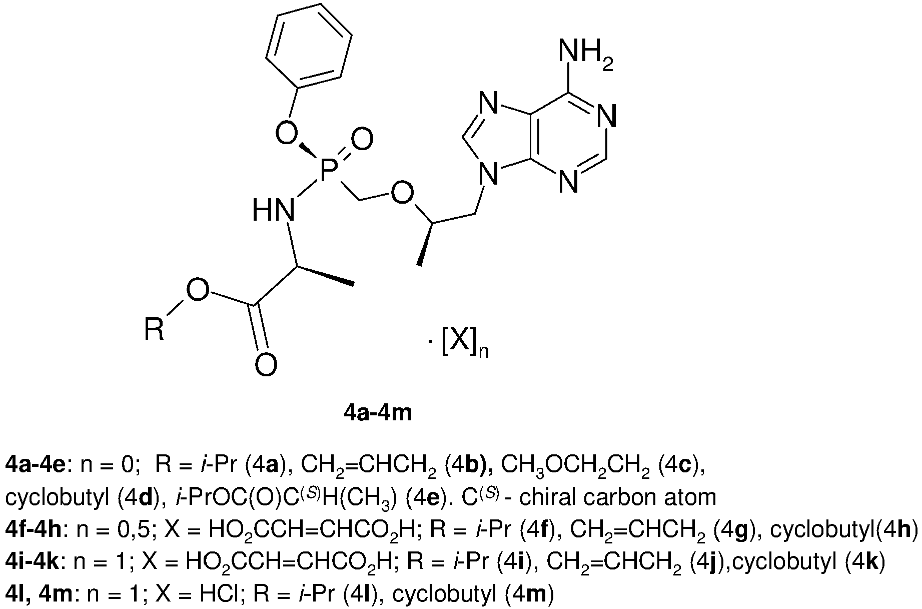

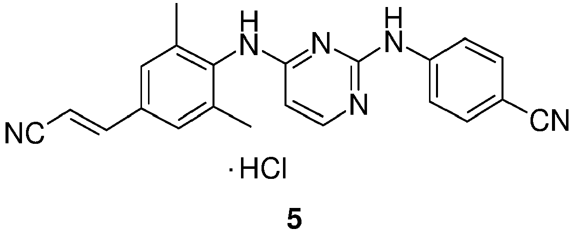

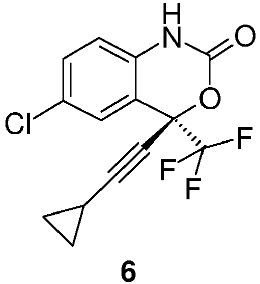

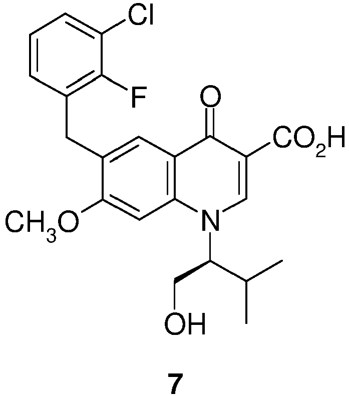

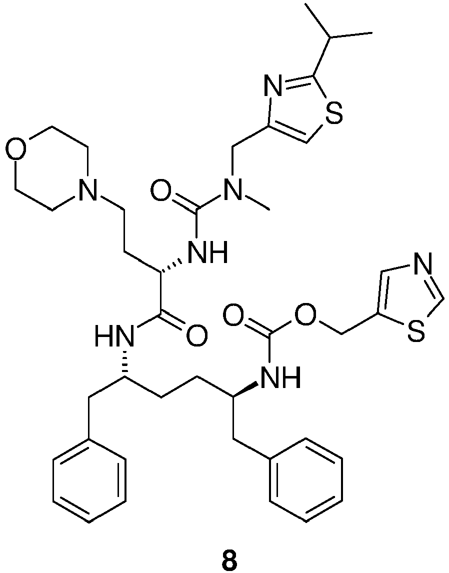

The drugs of formulas 1-8 have different mechanisms of action. Thus, elsulfavirine of formula 1, rilpirivine of formula 5, and efavirenz of formula 6 are non-nucleoside reverse transcriptase inhibitors (NNRTIs), the compounds of formulas 2a-2l are precursors of nucleoside reverse transcriptase inhibitors (NRTIs), and tenofovir of formulas 3, 4a-4m are precursors of nucleotide reverse transcriptase inhibitors (NtRTIs). Elvitegravir of formula 5 is an integrase inhibitor (INI), and cobicistat of formula 8 is a pharmacokinetic enhancer, a cytochrome P450 3A (CYP3A) inhibitor. It does not exhibit antiviral activity.

Examples of dual-component drugs include Truvada in tablets each comprising, as active ingredients, 200 mg of emtricitabine of formula 2b and 300 mg of tenofovir disoproxil fumarate of formula 3 [WO 2004064845. WO 2006135932. http://www.gilead.com/∼/media/files/pdfs/medicine s/hiv/truvada/truvada_pi_old.pdf], and Descovy, with each tablet comprising 200 mg of emtricitabine of formula 2b and 25 mg of tenofovir alafenamide hemifumarate of formula 4f [WO 2017004244. https://www.gilead.com/∼/media/files/pdfs/medicines/hiv/descov y/descovy_pi.pdf?la=en].

Today, routine treatment of viral diseases including HIV involves a combination of at least three drugs with different modes of action (said therapy is commonly called Highly Active Anti-Retroviral Therapy, or HAART) to suppress both HBV and HIV.

Combination drugs (tablets) comprising fixed API doses have proved to be especially convenient, safe, and efficient.

The most advanced drugs for HBV and HIV/AIDS HAART are

Eviplera (Complera) comprising the following APIs: 200 mg of emtricitabine of formula 2b, 245 mg of tenofovir disoproxil fumarate of formula 3, and 27.5 mg of rilpivirine hydrochloride of formula 5, [WO 2016005327. http://www.gilead.com/∼/media/files/pdfs/medicines/hiv/compler a/complera_pi_old.pdf];

Atripla comprising 200 mg of emtricitabine of formula 2b, 300 mg of tenofovir disoproxil fumarate of formula 3, and 600 mg of efavirenz of formula 6 [WO 2004064845. https://aidsinfo.nih.gov/drugs/424/atripla/0/patient/.https://mini-doctor.com/pilul/atripla_tabletki_pokritie_plenochnoy_obolochkoy_CO2HCCO2H_ vo_flakone-14866.html];

Genvoya comprising 200 mg of emtricitabine of formula 2b, 10 mg of tenofovir alafenamide hemifumarate of formula 4f, 150 mg of elvitegravir of formula 7, and 150 mg of cobicistat of formula 8 [https://www.gilead.com/∼/media/files/pdfs/medicines/hiv/genvoya/genvoya_pi.pdf ?la=en], and Odefsey comprising 200 mg of emricitabine of formula 2b, 25 mg of tenofovir alafenamide hemifumarate of formula 4f, and 25 mg of rilvipirine hydrochloride of formula 7 [WO 2017004244. [https://www.gilead.com/∼/media/files/pdfs/medicines/hiv/odefs ey/odefsey_pi.pdf?la=en].

The success of effective and well-tolerated HAART means that the morbidity and mortality rates among HIV-infected population are increasingly more often associated with concomitant diseases unrelated to AIDS. In clinical research, an increasingly greater focus is being put on the tolerance, long-term safety, and strict adherence to the ART procedure (Costagliola D. Demographics of HIV and aging. Curr. Opin. HIV AIDS, 2014, (4), 294). In this connection, there remains a significant medical need for novel effective and safe HAART techniques taking into account the age populations of patients, non-HIV related diseases, virological resistance and simplification of the treatment regimen.

### Summary of the invention

The inventors have found that a novel combination drug in a solid oral dosage form comprising, as one of the three active ingredients, elsulfavirine sodium of formula 1a can be used in medicine for treating viral infections including HIV and hepatitis B (HBV).

The subject matter of the present invention is a novel combination drug in a solid oral dosage form comprising, in a crystalline or polycrystalline form and in a therapeutically effective amount, elsulfavirine sodium of formula 1a, one of the NRTI precursors of formulas 2a-2j or a pharmaceutically acceptable salt thereof, and one of the NtRTIs precursors of formulas 3, 4a-4m or a pharmaceutically acceptable salt thereof optionally in combination with excipients (auxiliary agents).

Elsulfavirine of formula 1a is a prodrug of the active compound VM-1500A of formula 1b, which is a potent inhibitor of HIV-1 HXB2 strain replication in MT-4 cells and belongs to the class of Non-Nucleoside Reverse Transcriptase Inhibitors (NNRTIs). The mean value of IC50 obtained for VM-1500A of formula 1b on the HXB2wild-type strain and for inhibiting the replication of HIV-1 mutant viruses comprising mutations V106A, G190A, L100I/K103N, and K103N/Y181C are 1.3 ± 0.4 nM for HXB2, 1.2 ± 0.2 nM for V106A, 0.6 ± 0.6 nM for G190A, 1.3 ± 0.3 nM for L100I/K103N, and 1.3 ± 0.4 nM for K103N/Y181C.

NRTI precursors of formulas 2a-2I exhibit clinical activity against HIV and HBV [https://www.accessdata.fda.gov/drugsatfda_docs/label/2013/021003s015,021004 s015lbl.pdf; https://www.accessdata.fda.gov/drugsatfda_docs/label/2008/021500s010,021896s 004lbl.pdf; RU 2017106611] and are interest for ART.

Tenofovir disoproxil fumarate of formula 3 is an NtRTI precursor and has antiviral activity against HIV-1 and HBV [http://www.openaccessjournals.com/articles/tenofovir-disoproxil-fumarate-for-the-treatment-of-hepatitis-b-virus-infection-pharmacokinetics-and-clinical-efficacy.pdf].

Tenofovir of formula 4a-4m are also NtRTI precursors and are used for treating HIV infection [https://www.hepmag.com/article/fda-approves-vemlidy-tenofovir-alafenamide-taf-hepatitis-b] and chronic viral infection of hepatitis B.

Tenofovir of formula 4a-4e, 4g-4m are NtRTI precursors too, and, as the inventors have ascertained, they are active against HIV (Table 1) and HBV (Table 2) infections.

**Table 1. Anti-HIV activity (EC₅₀), cytotoxicity (CC₅₀), and selectivity index (SI) for tenofovir 4f, 4h, 4k, 4m**

| Compound | EC₅₀ (nM) | CC₅₀ (µM) | SI |
|---|---|---|---|
| 4f | 43.0 | >100 | >2.326 |
| 4h | 13.3 | >100 | >7.692 |
| 4k | 27.0 | >100 | >3.704 |
| 4m | 9.2 | 84.9 | 9.129 |

**Table 2. Anti-HBV activity (EC₅₀), cytotoxicity (CC₅₀), and selectivity index (SI) for tenofovir 4f, 4h, 4k, 4m**

| Compound | EC₅₀ (nM) | CC₅₀ (µM) | SI |
|---|---|---|---|
| 4f | 0.7 - 0.3 | 3 | 4286-10000 |
| 4h | 0.75 | 6 | 8000 |
| 4k | 3.6 | 2.7 | 750 |
| 4m | 0.04 | >10* | >133333 |

| | | | |
|---|---|---|---|
| *70% viable cells at 10 µM | | | |

The novel combination drug in a solid oral dosage form is safe and simplifies the therapeutic regimen. In addition, owing to elsulfavirine sodium of formula 1a, it shows efficacy in the treatment of viral diseases being active against HBV and HIV wild and mutant viruses.

Listed below are definitions of various terms used to describe this invention. These definitions apply to the terms as they are used throughout this specification and claims, unless otherwise limited in specific instances, either individually or as part of a larger group.

The term "active ingredient" (drug substance) refers to a physiologically active compound of synthetic or other origins that exhibits pharmacological activity and is an active ingredient of a pharmaceutical composition.

The term "inert filler" as used herein refers to a compound that is used for forming a pharmaceutical composition and is, as a rule, safe, nontoxic, and neither biologically nor otherwise undesirable and comprises excipients acceptable for veterinary and human pharmaceutical use. Compounds of this invention may be administered individually but are generally administered in a mixture with one or more pharmaceutically acceptable excipients, diluents, or carriers chosen depending on the contemplated route of drug administration and standard pharmaceutical practice.

The term "combination drug" refers to an oral solid dosage form or a pharmaceutical composition in tablets, gelatin capsules, pills, powders, and chewing gums to be used for the treatment and prophylaxis of viral diseases.

The term "crystalline form" refers to a substance structure wherein the molecules are arranged to form a crystal lattice.

The terms "treatment" and "treatment of the disease" include:
(1) prevention or reduction of the risk of disease development, i.e., prevention of the development of clinical symptoms in a subject who may be susceptible or predisposed to the disease but does not yet experience or show symptoms of the disease;
(2) inhibition of the disease, i.e., stoppage or subsidence of disease progression or clinical symptoms; and
(3) alleviation of the disease, i.e., inducing regression of the disease or its clinical symptoms.

The term "mass/mass percent" refers to the mass of a component expressed as a percentage of the total mass, for example, the layer of a substance or a dosage form comprising said component. Thus, a composition comprising "5 mass/mass percent of X" refers to a composition wherein the weight of component X makes up 5% of the total mass of the composition.

The term "polycrystalline form" refers to a polycrystalline substance structure consisting of a plurality of small monocrystals, or crystallites of certain crystalline form.

The term "subject" refers to a mammal, including, but not limited to, cattle, pigs, sheep, chickens, turkeys, buffalos, lamas, ostriches, dogs, cats, and humans, with humans being preferable.

The term "segregation" as used herein refers to certain components (for example, A and B) in a tablet and means that said components are physically discrete and the presence of one component (for example, A) does not significantly affect the storage stability of another component (or components) (for example, B), from which said component is separated. As a rule, when components are separated in the tablet, they will be present in individual layers of a multilayered tablet. For example: components A and B can be present in individual layers of a multilayered tablet, wherein the layer (a) comprising component A is basically free from component B and the the layer (b) comprising component B is basically free from component A. Individual layers may be either in contact with each other or separated by, for example, one or more additional layers.

The term "comprise" and variations thereof, such as "comprises" or "comprising," are to be construed in an open, inclusive sense and mean "including, but not limited to". The term "between" in relation to two values includes both these values, for example, the range "between" 10 mg and 20 mg covers 10, 11, 12, 13, 14, 15, 16, 17, 18, 19, and 20 mg.

The term "solvate" refers to a molecular complex comprising a compound and one or more pharmaceutically acceptable molecules of a solvent. Examples of solvent molecules include water and C₁-C₆ alcohols like ethanol. When the solvate means water, the term "hydrate" can be used.

The term "therapeutically effective amount," as used herein, refers to an amount of a substance, prodrug, or drug needed for alleviating the symptoms of the disease in the subject. The dose of a substance, prodrug, or drug should meet individual demands in each particular case. Said dose may vary in a wide range depending on numerous factors like the severity of the disease to be treated, the age and the general condition of the patient, other medicaments used for the patient's treatment, the mode and route of administration, and the experience of the attending doctor. For oral administration, the daily dose is approximately 0.01-10 g, including all values there between, both in monotherapy and/or combination therapy. The preferred daily dose is around 0.1-7 g. As a rule, in order to quickly alleviate or eliminate the virus, a higher loading dose is given at the beginning of treatment with a subsequent reduction of the dose to a level sufficient to prevent an infection burst.

The term "pharmaceutical composition" refers to a composition comprising active ingredients and optionally at least one component selected from the group consisting of pharmaceutically acceptable and pharmacologically compatible inert fillers, solvents, diluents, carriers, excipients, distributing and delivery agents such as preservatives, stabilizers, fillers, disintegrators, moisteners, emulsifiers, suspending agents, thickeners, sweeteners, flavoring and antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof. Protection against microorganisms can be provided using various antibacterial and antifungal agents, such as parabens, chlorobutanol, sorbic acid, and the like. Said composition may also comprise isotonic agents, such as sugar, sodium chloride, and the like. The sustained action of the composition can be achieved using agents that decelerate the absorption of the active ingredient, for example, aluminum monostearate and gelatin. Examples of suitable carriers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight.

The term "pharmaceutically acceptable" refers to those compounds, which are, within the scope of sound medical judgment, safe and suitable for use, without excessive toxicity, irritation, allergic response or other complications commensurate with a reasonable benefit/risk ratio.

The term "pharmaceutically acceptable salt" refers to a salt of a pharmaceutically acceptable compound that possesses (or can be modified into a form that possesses) a desirable pharmacological activity of the parent compound. Said salts include acid addition salts derived from inorganic acids, such as hydrochloric, hydrobromic, sulfuric, nitric, phosphoric, and the like; or salts derived from organic acids, such as acetic, benzenesulfonic, benzoic, camphorsulfonic, citric, ethanesulfonic, fumaric, glucoheptonic, gluconic, lactic, maleic, malonic, mandelic, methanesulfonic, 2-naphthalenesulfonic, oleic, palmitic, propionic, stearic, succinic, tartaric, π-toluenesulfonic, pivalic, and the like, and salts formed when the acidic proton present in the parent compound is substituted either by a metal ion, for example, an alkali metal ion or an aluminum ion, or organic base salts, such as diethanoloamine, triethanolamine, N- methylglucamine, and the like. This definition also covers ammonium and substituted or quaternary ammonium salts. Typical non-limiting lists of pharmaceutically accepted salts can be found in S.M. Berge et al., J. Pharm Sci., 66 (1), 1-19 (1977) and Remington: Science and Practice of Pharmacy, R. Hendrickson, 21st ed., Lippincott, Williams & Wilkins, Philadelphia, PA, (2005), at p. 732, Table 38-5, both of which are incorporated herein by reference.

The subject matter of the present invention is a novel combination drug in a solid oral dosage form comprising, as one of the three active ingredients, a therapeutically effective amount of elsulfavirine sodium of formula 1a in a crystalline or polycrystalline form, optionally in combination with excipients (auxiliary substances).

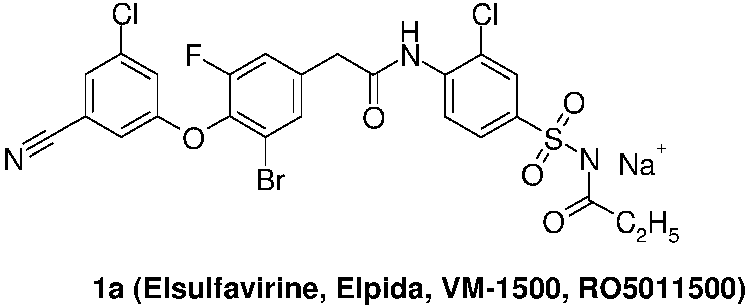

More preferable is a combination drug in a solid oral dosage form comprising, in a crystalline or polycrystalline form in a therapeutically effective amount, elsulfavirine sodium of formula 1a, one of the NRTI precursors of formulas 2a-2j or a pharmaceutically acceptable salt thereof and one of the NtRTI precursors of formulas 3, 4a-4m or a pharmaceutically acceptable salt thereof optionally in combination with excipients (auxiliary substances).

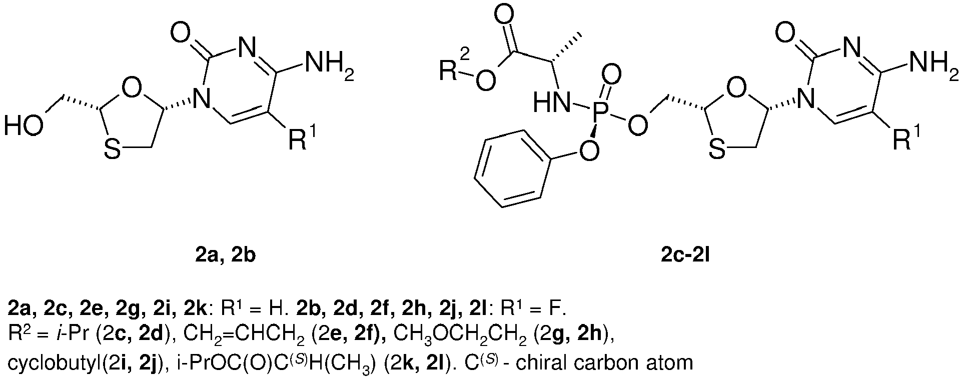

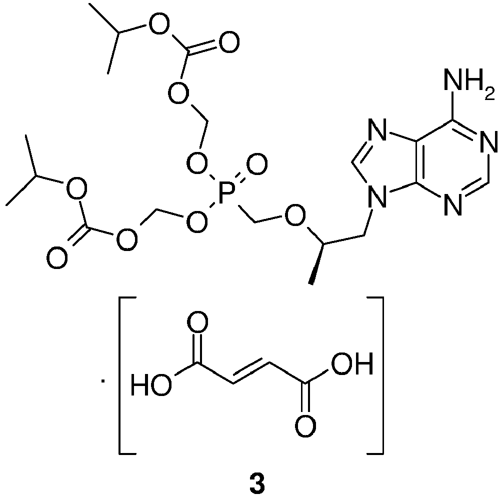

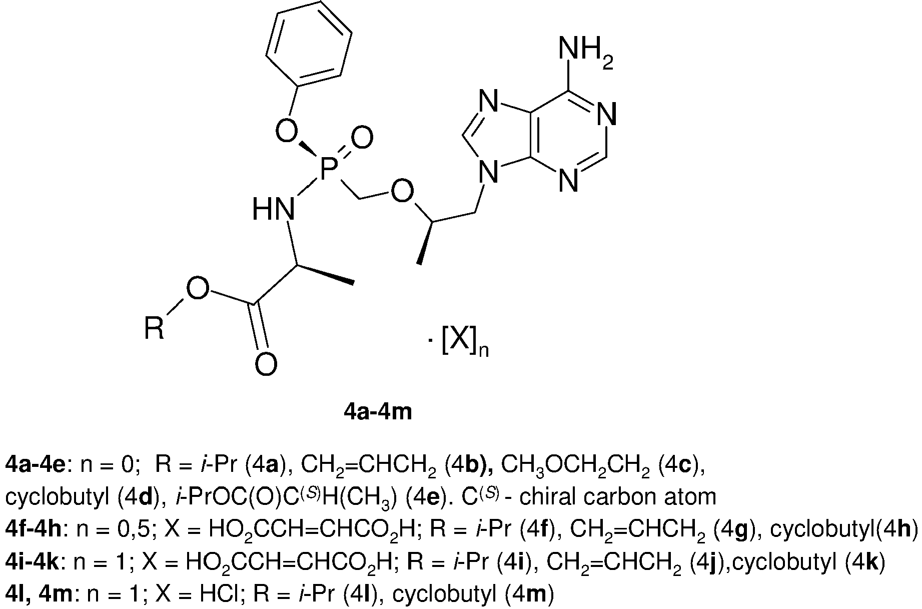

A preferable embodiment of the present invention is a combination drug in a solid oral dosage form in tablets, gelatin capsules, pills, powders, granules, or medicated chewing gums.

A preferable embodiment of the present invention is a combination drug in a solid oral dosage form in tablets, gelatin capsules, pills, powders, granules, or medicated chewing gums comprising, as active ingredients, elsulfavirine sodium of formula 1a, an NRTI precursor of formulas 2a-2j or a salt thereof and tenofovir disoproxil fumarate of formula 3 in a mass ratio of (**1a**) : (**2a-2j**) or a salt thereof: (**3**) ≈ 1:10:15.

More preferable is also a combination drug in a solid oral dosage form in tablets, gelatin capsules, pills, powders, granules, or medicated chewing gums comprising, as active ingredients, 15-25 mg of elsulfavirine sodium of formula 1a, 150-300 mg of an NRTI precursor of formulas 2a-2j or a salt thereof, and 250-350 mg of tenofovir disoproxil fumarate of formula **3.**

More preferable is also a combination drug in a solid oral dosage form in tablets comprising, as active ingredients: elsulfavirine sodium of formula **1a**, an NRTI precursor of formulas **2a-2j,** and tenofovir disoproxil fumarate of formula **3;** as excipients: lactose monohydrate 200, microcrystalline cellulose 102, croscarmellose sodium, pre-gelled starch, Povidone K30, and magnesium stearate; and, as a film coating, Vivacoat PC-8T-181, with the mass ratio depending on their nature and mode of production.

More preferable is also a combination drug in a solid oral dosage form in tablets comprising, as active ingredients, 20,7 mg of elsulfavirine sodium of formula **1a,** 200-300 mg of an NRTI precursor of formulas **2a-2j,** 300 mg of tenofovir disoproxil fumarate of formula **3,** 386.9 mg of lactose monohydrate 200, 134.2 mg of microcrystalline cellulose 102, 67.1 mg of croscarmellose sodium, 33.4 mg of pre-gelled starch, 15.0 mg of Povidone K30, 10.7 mg of magnesium stearate, and, as a film coating, 50.0 mg of Vivacoat PC-8T-181.

Another embodiment of the present invention is a combination drug in a solid oral dosage form in tablets, gelatin capsules, pills, powders, granules, or medicated chewing gums comprising, as active ingredients, elsulfavirine sodium of formula 1a, an NRTI precursor of formulas 2a-2j or a salt thereof, and tenofovir of formulas 4a-4m in a mass ratio of (1a) : (2a-2j) or a salt thereof: (4a-4m) ≈ 1:10:1,25.

A more preferable embodiment of the present invention is a combination drug in a solid oral dosage form in tablets, gelatin capsules, pills, powders, granules, or medicated chewing gums comprising, as active ingredients, 15-25 mg of elsulfavirine sodium of formula 1a, 150-300 mg of an NRTI precursor of formulas 2a-2j or a salt thereof, and 10-35 mg of tenofovir of formulas **4a-4m.**

A more preferable embodiment of the present invention is a combination drug in a solid oral dosage form in tablets, gelatin capsules, pills, powders, granules, or medicated chewing gums comprising, as active ingredients, 15-25 mg of elsulfavirine sodium of formula **1a,** 150-300 mg of an NRTI precursor of formulas **2a-2j** or a salt thereof, and 10-35 mg of tenofovir of formula **4h** or **4m.**

More preferable is also a combination drug in a solid oral dosage form in tablets comprising, as active ingredients: 15-25 mg of elsulfavirine sodium of formula **1a,** 150-300 mg of an NRTI precursor of formulas **2a-2j** or a salt thereof, and 10-35 mg of tenofovir of formulas **4a-4m**); as excipients (auxiliary agents): lactose monohydrate 200, microcrystalline cellulose 102, croscarmellose sodium, pre-gelled starch, Povidone K30, and magnesium stearate; and, as a film coating, Vivacoat PC-8T-181, with the mass ratio depending on their nature and mode of production.

More preferable is also a combination drug in a solid oral dosage form in tablets comprising, as active ingredients 20.7 mg of elsulfavirine sodium of formula **1a,** 200-300 mg of an NRTI precursor of formulas **2a-2j,** 25 mg of tenofovir of formula **4f, 4h** or **4m,** 386.9 mg of lactose monohydrate 200, 134.2 mg of monocrystalline cellulose 102, 67.1 mg of croscarmellose sodium, 33.4 mg of pre-gelled starch, 15.0 mg of Povidone K30, and 10.7 mg of magnesium stearate; and, as a film coating, 50.0 mg of Vivacoat PC-8T-181.

More preferable is also a combination drug in a solid oral dosage form in tablets comprising, as active ingredients, 10-25 mg of elsulfavirine sodium of formula **1a,** 150-350 mg of an NRTI precursor of formulas **2a-2j,** 5-35 mg of tenofovir of formula **4f, 4h** or **4m,** 20-35 mg of croscarmellose sodium, 70-120 mg of microcystalline cellulose, and 1-7 mg of magnesium stearate.

More preferable is also a combination drug in a solid oral dosage form in tablets comprising, as active ingredients, 20.7 mg of elsulfavirine sodium of formula **1a,** 200-300 mg of an NRTI precursor of formulas **2a-2j,** 25 mg of tenofovir of formula **4f, 4h** or **4m,** 28 mg of croscarmellose sodium, 105.56 mg of cellulose microcrystals, 5.25 mg of magnesium stearate, and a film coat consisting of Vivacoat PC-8T-181.

Further embodiment of this invention is a kit comprising a combination drug in an oral solid dosage form in tablets, gelatin capsules, pills, powders, granules, or medicated chewing gums and a desiccant, preferably, silica gel.

Another embodiment of this invention is a method for producing a combination drug in a solid oral dosage form consisting of three active ingredients made in tablets, gelatin capsules, pills, powders, granules, or medicated chewing gums as described above.

Another embodiment of this invention is a method for producing a combination drug in tablets by mixing therapeutically effective amounts of elsulfavirine sodium of formula 1a, an NRTI precursor of formulas **2a-2j** or a pharmaceutically acceptable salt thereof, and an NtRTI precursor of formula **3** or formulas **4a-4m** or a pharmaceutically acceptable salt thereof with excipients followed by compression.

Another embodiment of this invention is a dry granular mixture of elsulfavirine sodium of formula **1a,** an NRTI precursor of formulas **2a-2j** or a pharmaceutically acceptable salt thereof, and tenofovir of formula **3, 4f, 4h** or **4m.**

Another embodiment of this invention is a combination drug in a solid oral dosage form made in tablets, gelatin capsules, pills, powders, granules, or medicated chewing gums to be used for the medical treatment of viral infection.

Another embodiment of this invention is a combination drug in a solid oral dosage form made in tablets, gelatin capsules, pills, powders, granules, or medicated chewing gums to be used for the medical treatment of HIV infection.

Another embodiment of this invention is a combination drug in a solid oral dosage form made in tablets, gelatin capsules, pills, powders, granules, or medicated chewing gums to be used for the medical treatment of hepatitis B (HBV).

Another embodiment of this invention is a method of medical treatment of HIV infection comprising administration to the subject in need thereof of the described above novel combination drug in a solid oral dosage form made in tablets, gelatin capsules, pills, powders, granules, or medicated chewing gums.

Another embodiment of this invention is a HIV prevention method comprising administration to the patient of a combination drug in a solid oral dosage form.

Another embodiment of this invention is a method, wherein a combination drug in a solid oral dosage form can be administered less than once daily.

Another embodiment of this invention is a method, wherein a combination drug in a solid oral dosage form is administered to the subject prior and after the event increasing the risk of HIV contamination.

The present invention relates to a tablet comprising elsulfavirine sodium of formula **1a,** an NRTI precursor of formulas **2a-2j** or a salt thereof, and tenofovir of formula **3, 4f, 4h** or **4m.**

Said tablet has a coating, preferably, a film coating like Vivacoat PC-8T-181.

The tablet preferably comprises 20.7 mg of elsulfavirine sodium of formula **1a,** 200-300 mg of an NRTI precursor of formulas **2a-2j** or a salt thereof, and 300 mg of tenofovir disoproxil fumarate of formula **3.**

The tablet preferably comprises 20.7 mg of elsulfavirine sodium of formula **1a,** 200-300 mg of an NRTI precursor of formulas **2a-2j** or a salt thereof, and 25 mg of tenofovir of formula **4f, 4h** or **4m.**

The tablet preferably comprises the following excipients: lactose monohydrate 200, microcrystalline cellulose 102, croscarmellose sodium, pre-gelled starch, Povidone K30, magnesium stearate, and, as a film coating, Vivacoat PC-8T-181.

The tablet according to the invention preferably has a total mass of 1268 mg ± 500 mg, or 1268 ± 200 mg, or 1268 ± 50 mg, or 1268 mg.

The tablet preferably comprises a film coating preferably containing polyvinyl alcohol, polyethylene glycol, talc, titanium dioxide, and black iron oxide.

The tablet preferably comprises a film coating consisting of 50 mg of Vivacoat PC-8T-181.

In one embodiment of this invention, from 29 %wt to 68 %wt of the tablet is elsulfavirine sodium of formula 1a, an NRTI precursor of formulas 2a-2j or a salt thereof, and tenofovir disoproxil fumarate of formula 3.

In another embodiment of this invention, from 19 %wt to 50 %wt of the tablet is elsulfavirine sodium of formula **1a,** an NRTI precursor of formulas **2a-2j** or a salt thereof, and tenofovir of formula **4f, 4h** or **4m.**

In another embodiment of this invention, from 1.1 %wt to 2.7 %wt of the tablet is elsulfavirine sodium of formula **1a,** from 11.3 %wt to 39.1 %wt of the tablet is an NRTI precursor of formulas **2a-2j** or a salt thereof, and from 16.9 %wt to 39.1 %wt of the tablet is tenofovir disoproxil fumarate of formula **3.**

In another embodiment of this invention, from 1.1 %wt to 2.7 %wt of the tablet is elsulfavirine sodium of formula **1a**, from 11.3 %wt to 39.1 %wt of the tablet is an NRTI precursor of formulas **2a-2j** or a salt thereof, and from 1.4 %wt to 3.3 %wt of the tablet is tenofovir of formula **4f, 4h** or **4m.**

The subject matter of the present invention is a method for producing a combination drug in a solid oral dosage form, particularly a tablet, said method consisting in mixing active ingredients with excipients (auxiliary agents) and subsequent compressing. In some embodiments, active ingredients are first mixed and granulated with excipients (auxiliary agents), for example, by way of dry granulation. In some embodiments, this stage includes roller compaction and/or grinding. In other embodiments, granulated mixed active ingredients are additionally combined with extragranular excipients (auxiliary agents) and then compressed.

In some embodiments, said method involves separate compression of three active ingredients to produce a three-layer tablet.

As a rule, the methods comprise a tablet core coating stage following compression with, for example, a film coating as described above.

Tableting methods are generally well known in pharmaceutics and described in a popular book [Remington's Pharmaceutical Sciences, 17th ed. Edited by Alfonso R. Gennaro. Mack Publishing Co., 20th and Northampton Streets, Easton, PA 18042. 1985].

The tablet can be produced by way of pressing or moulding, optionally with one or more fillers. Compressed tablets can be produced by pressing loose active ingredients like powder or granules optionally mixed with fillers in a suitable machine.

The novel combination drug in a solid oral dosage form, more particularly, a tablet, can be used for treating or preventing viral diseases, including for treating and preventing hepatitis B (HBV), HIV-1, or HIV-2.

Another subject matter of this invention is a method for treating viral infections in subjects (patients), including HBV- and HIV-infected ones, by oral administration to the patient of the novel combination drug in a solid oral dosage form, more particularly, a tablet.

Further subject matter of the invention is a method to prevent HBV and HIV-infection in HBV- and HIV-exposed subjects by oral administration to the patient of the novel combination drug in a solid oral dosage form, more particularly, a tablet.

The methods of HBV and HIV prophylaxis and treatment disclosed herein comprise administration of the novel oral dosage form disclosed herein (in particular, a tablet) to the subject, commonly a human, and will usually comprise repeated administrations, commonly once daily.

In some embodiments of this invention, the methods disclosed herein comprise repeated administrations at intervals less than once a day. For example, in some embodiments the methods disclosed herein comprise administration of novel oral dosage forms disclosed herein every other day, five times a week, four times a week, three times a week, twice a week, or once a week.

In some embodiments of this invention, the methods disclosed herein comprise administration before and/or after the event that may expose the individual to HBV and HIV, or would otherwise increase the risk of HIV infection by the individual, for example, for pre-exposure prophylaxis and/or for post-exposure prophylaxis. Examples of events that may increase the risk of HIV contamination include, without limitation, condom use at having anal sex with an HBV- or HIV-positive partner or a partner with unknown HBV or HIV status; anal sex with more than 3 intercourse partners; sex with a male partner diagnosed with sexually transmitted infections; and failure to use condoms consistently with a sexual partner who is known to be HBV- or HIV-positive.

In some embodiments, for example, administration for pre-exposure prophylaxis, novel solid oral dosage forms disclosed herein are administered from 2 to 72 hours, from 2 to 48 hours, from 2 to 24 hours, or from 2 to 12 hours prior to the event increasing the risk of contamination (for example, prior to a sexual intercourse or other exposure to HBV or HIV). In some embodiments, novel solid oral dosage forms disclosed herein are administered within 72 hours, 60 hours, 48 hours, 24 hours, 12 hours, 9 hours, 6 hours, 4 hours, 3 hours, 2 hours, or 1 hour prior to the event increasing the risk of HIV contamination (for example, prior to a sexual intercourse or other exposure to HBV or HIV). In some embodiments, when novel solid oral dosage forms disclosed herein are administered prior to the event increasing the risk of HBV or HIV contamination, said dosage forms are administered every day prior to the event. In some embodiments, when solid oral dosage forms disclosed herein are administered prior to the event increasing the risk of HBV or HIV contamination, said dosage forms are administered from one to three times prior to the event.

In some embodiments, for example, when using novel solid oral dosage forms disclosed herein as part of the preventive regimen, said dosage forms are administered from 2 to 48 hours, from 2 to 36 hours, from 2 to 24 hours, or from 2 to 12 hours after the event increasing the risk of HIV contamination (for example, following the sexual intercourse or other exposure to HIV). In some embodiments, for example, when using novel solid oral dosage forms disclosed herein for prophylaxis, said dosage forms are administered within 7 days, 14 days, 21 days, 28 days, 30 days, or 45 days after the event increasing the individual risk of HIV contamination (for example, following the sexual intercourse or other exposure to HBV or HIV). In some embodiments,, for example, when using novel solid oral dosage forms disclosed herein for prophylaxis, said dosage forms are administered within 30 days after the event increasing the individual risk of HBV or HIV contamination (for example, following the sexual intercourse or other exposure to HBV or HIV). In some embodiments, solid oral dosage forms described herein are administered within less than 1 hour, 2 hours, 3 hours, 4 hours, 5 hours, 6 hours, 7 hours, 8 hours, 9 hours, 12 hours, 18 hours, 24 hours, 36 hours, or 48 hours after the event that would increase the risk of contamination (for example, following the sexual intercourse or other exposure to HBV or HIV). In some other embodiments, solid oral dosage forms disclosed herein are administered within 1 day, 2 days, 3 days, 4 days, or 5 days after the event that would increase the risk of contamination (for example, following the sexual intercourse or other exposure to HBV and HIV). In some embodiments, when novel solid oral dosage forms disclosed herein are administered after the event that would increase the risk of HBV or HIV contamination, said dosage forms are prescribed on a daily basis. In some embodiments, when novel solid oral dosage forms disclosed herein are administered after the event that would increase the risk of HBV or HIV contamination, said dosage forms are administered from one to three times after the event. In some embodiments, when solid oral dosage forms disclosed herein are administered after the event that would increase the risk of HBV or HIV contamination, said dosage forms are administered once after the event.

### Preferred embodiment

The present invention will now be described in terms of certain embodiments, which are not intended to limit its scope. On the contrary, the present invention covers all alternatives, modifications, and equivalents that can be included within the scope of the claims. Thus, the following examples, which include specific embodiments, will illustrate this invention without limiting it.

### Example 1. General procedure for preparing Solid Oral Dosage Forms (SODFs).

Elsulfavirine sodium of formula **1a** (2.0-2.5 g), an NRTI precursor of formulas **2a-2j** (20.0-30.0 g), and tenofovir disoproxil fumarate of formula **3** (30.0 g) or tenofovir of formulas **4a-4m** (15-35 mg) are carefully ground and mixed. The resulting SODF (Table 3) is used to produce a combination drug in tablets, gelatin capsules, pills, powders, granules, or medicated chewing gums by known process.

**Table 3. The content of active ingredients in SODF**

| Ingredient | Solid Oral Dosage Forms (SODFs) | | | | | | |
|---|---|---|---|---|---|---|---|
| | SODF 1 | SODF 2 | SODF 3 | SODF 4 | SODF 5 | SODF 6 | SODF 7 |
| | Content of active ingredients (g) in SODF | | | | | | |
| **1a** | 2.07 | 2.07 | 2.07 | 2.07 | 2.07 | 2.07 | 2.07 |
| **2b** | 20.0 | | 20.0 | | | 20.0 | 20.0 |
| **2j** | | 25.0 | | 25.0 | 25.0 | | |
| **3** | 30.0 | 30.0 | | | | | |
| **4f** | | | 2.5 | | | | |
| **4h** | | | | 2.5 | | 2.5 | |
| **4m** | | | | | 2.5 | | 2.5 |

### Example 2. Evaluation of anti-HIV activity, cytotoxicity, and selectivity index for tenofovir of formulas 4f, 4h, 4k, and 4m.

The anti-HIV activity of active ingredients (test compounds) was evaluated using SupT1 cells. The cells were infected with the NL4.3 HIV strain carrying a gene of green fluorescence protein (NL4.3-GFP). The virus preparation was obtained by transfection of 293T cells of proviral DNA. Forty-eight hours after the transfection, the preparation was frozen and stored until use. To enhance the infection efficiency, the suspension of SupT1 cells was precipitated from the infection mixture by centrifugation. Test compounds were added to the cells immediately before virus addition. After a 2-hour incubation, the infection mixture was replaced by fresh culture medium with test compounds. The infection efficiency was evaluated following 45 hours by counting the percent of fluorescing cells against noninfected cell cultures. The cytotoxicity of test compounds was evaluated simultaneously at the same, but not infected, SupT1 cellular line using the XTT reagent. To this end, serial ten-fold dilutions of test compounds were prepared (starting with 10 µM to evaluate viral activity or with 100 µM to evaluate cytotoxicity). For negative control, 0.1% DMSO was used. As a result, the values of activity EC₅₀, cytotoxicity CC₅₀, and selectivity index SI were calculated. The test quality was evaluated using the following controls: signal to background ratio, integrase inhibitor raltegravir (1 µM), and reproducibility of the test. The drug emetine was used to control the validity of cytotoxicity obtained (0.03, 0.09, and 0.2 µM). The results are summarized in Table 1.

### Example 3. Evaluation of anti-HBV activity, cytotoxicity, and selectivity index for tenofovirs of formulas 4f, 4h, 4k, 4m.

Anti-HBV activity of tenofovir of formulas **4f, 4h, 4k, 4m** (test compounds) was evaluated in the cell line of human hepatoma AD38 carrying integrated HBV DNA with terminal repeats [Lander S, et. al, Antimicrobial Agents and Chemotherapy, 1997, pp. 1715-1720]. The cell line was made available by Dr. C. Seeger, Fox Chase Cancer Center, Philadelphia, PA). Simultaneously, cytotoxicity was evaluated.

The cells were cultivated in complete DMEM/F12 culture medium containing 2 mM of L-glutamine (Thermo Scientific, Cat #11320033), 10% fetal bovine serum (ThermoFisher Scientific, Cat#), 1% antibiotic-antimycotic solution (ThermoFisher Scientific, Cat#15240096), and 0.3 µg/ml of tetracycline (Sigma, Cat # T7660-5G). The cells were seeded into 96-well Corning Biocoat plates (Corning, Cat # 356407) in 225 µl of complete medium without tetracycline, 20 000 cells per well. The test compounds were dissolved first in DMSO (Sigma cat. D2650), then in DMEM/F12 medium, and 9 dilutions in log 3 steps (225 µl) were added to the cells. The final concentrations of test compounds varied from 10 µM to 1 nM. Each dilution was tested in three identical wells. For inhibition control, cells cultivated in the presence of tetracycline were used, because tetracycline completely stops HBV replication in this cell line. The cells were then incubated for 4 days under a humidified 5% CO₂ atmosphere at 37°C.

Isolation of secreted HBV DNA. Following a 4-day incubation, the viral DNA was isolated from cultural supernatants using the PureLink® Pro 96 Genomic DNA Purification Kit (ThermoFisher Scientific, Cat # K183104A) according to the manufacturer's instructions. After elution, the purified DNA was stored at -20°C.

The technique of real-time quantitative polymerase chain reaction (RT-qPCR) was applied using the CFX96TM Real-Time System instrument (Bio-Rad, Hercules, CA) and polymerase AmpliTaq Gold® DNA Polymerase (Applied Biosystems®).

**Reaction mixture composition:**

| | |
|---|---|
| 10X reaction buffer | 2 µl |
| 25 mM MgCl₂ | 2 µl |
| dNTP mix (ATP,GTP,CTP - 2 mM, UTP - 4 mM) | 2 µl |
| ROX, 50 µM (Fisher Scientific117545000) | 0.04 µl |
| 20X primers/probe mix (primers 6 µM, probe 5 µM) | 1 µl |
| Uracil-DNA Glycosylase (1 U/µl) | 0.15 µl |
| AmpliTaq Gold® DNA Polymerase (5U/µl) | 0.1 µl |
| Water | 12.11 (up to 19.4 µl) |
| DNA HBV | 0.6 µl |

**Cycle program:**

| | |
|---|---|
| 50°C | 2 minutes |
| 95°C | 10 minutes |

| 40 cycles: | |
|---|---|
| 95°C | 15 seconds |
| 60°C | 30 seconds |
| 72°C | 30 seconds + 1 second per each subsequent cycle. |

The fluorescent signal was read at the end of each cycle.

Primers and fluorescent samples were obtained from IDT (San Diego, CA):
HBV_rcDNA-S_FAM
56FAM/ATCCTCAAC/ZEN/CACCAGCACGGGACCA/3IABkFQ;
HBV_rcDNA-S_R GAGGGATACATAGAGGTTCCTTGA;
HBV_rcDNA-S_F GTTGCCCGTTTGTCCTCTAATTC.

The values of Ct ("threshold cycle," in which HBV DNA amplification becomes appreciable) normalized to cell cultures without test compounds were found by using the formula E = (1/(1+100%))^{∧}(Ct[test compound]-Ct[K-]), where E is a normalized level of HBV DNA, Ct[K-] and Ct[test compound] are Ct values for samples without and with test compounds, respectively. EC₅₀ values (Table 2) were computed using the Graph Prizm software.

The cytotoxicity of test compounds was determined simultaneously on the same AD38 cell line. The cells were cultivated in a 96-well black plate with a transparent bottom (104 cells/well) in complete DMEM/F12 medium containing 2 mM of L-glutamine (Thermo Scientific, Cat #11320033), 10% fetal bovine serum (ThermoFisher Scientific, Cat#), and 1% antibiotic-antimycotic solution (ThermoFisher Scientific, Cat#15240096). The AD38 cells were seeded in 96-well plates (7.5x103 cells/well in 100 µl of medium). The solutions of test compounds in DMEM were prepared immediately before use. All in all, there were 9 serial 3-fold dilutions. Four hours following cell seeding, the serial dilutions of preparations were added to the cells (100 µl per well). The final concentration of test compounds varied from 30 µM to 10 nM and that of DMSO, 0.5%. Whenever necessary, higher concentrations of test compounds were studied. The cells were then incubated for 3 days under a humidified 5% CO₂ atmosphere at 37°C. The number of living cells was counted using the ATPLite kit (Perkin Elmer, Boston, USA) in compliance with manufacturer's instructions. Three independent repeats were provided for each compound. Each cell was washed three times with PBS (0.2 ml/well) and then lysed by adding cell buffer (50 µl/well). All reagents mentioned hereinabove were included in the ATPLite kit. The microplate was incubated for 5 minutes on a rocking platform at 600 rev/min, following which a substrate solution (part of the ATPLite kit) was added (50 µl/well). After additional 5-minute incubation on a rocking platform at 600 rev/min, the plates were kept in dark for 10 minutes, and the luminescence was read using TopCount NXT (Packard, Perkin Elmer). For quantitative evaluation of cytotoxicity, the CC₅₀ parameter (a concentration that kills 50% of cells) was used. Calculation of CC₅₀: inhibition efficacy (% Inh) was calculated by using the formula: % Inh = [(Lpos - Lex)/ Lpos - Lneg)] ^{∗} 100%, where Lpos is positive control, luminescence in wells containing cells without compound medium; Lneg is negative control, luminescence in wells containing medium without cells; Lex is luminescence in wells containing medium in a particular concentration. CC₅₀ values (Table 2) were then determined using XLfit 4 software.

### Example 4. Evaluation of acute toxicity and tolerated toxic and lethal doses of SODFs.

Evaluation of SODF acute toxicity and tolerated toxic and lethal doses for single intragastric administration to male and female mice and rats. Evaluation was carried out for 24 male rats weighing 235-260 g and 28 female rats weighing 225-250 g as well as for 24 male mice weighing 21-25 g and 24 female mice weighing 20-24 g. All in all, there were 8 groups in each of the four categories. SODF was administered in the largest possible volume 10 ml/kg three times a day at a 40-minute interval. Prior to administration, SODFs were dispersed, then ground in a mortar and mixed with a 0.5% Tween 80 solution to obtain a suspension suitable for intragastric administration to animals at a dose of ≤ 10 ml/kg. Solutions for administration were always prepared on the day of administration. Prepared SODF suspensions were administered at the same time every day (within a deviation of maximum 4 hours). Prior to SODF administration, animal weights were recorded; after administration, experimental animals' condition was observed for 1 hour. On each of the 14 days following SODF administration, the weights of rats and mice were recorded and the animals were examined to detect any cases of death or abnormal condition. In this way, parameters of acute SODF toxicity were established. It was technically impossible to estimate LD50 for SODF, as the administration of maximum permissible SODF doses did not lead to animal death. Thus, it was found that intragastric SODF administration at doses like 600/6000/9000 mg/kg did not affect body weight gain just like in groups receiving reference drugs (compound of formula **1a**) at a dose of 3000 mg/kg and Truvada (compound of formula **2b** + compound of formula **3**) at a dose of 6000/9000 mg/kg. The latter did not differ from control or experimental groups of mice or rats (both males and females). In addition, no reduced feed or water intake was observed in all experimental and control groups. The intragastric administration of SODF and reference drugs did not affect the relative weight of animals' organs in experimental groups, and no statistically significant difference with control groups was detected. The gross examination of animals treated with drugs did not reveal any differences from control groups.

### Example 5. A SODF combination drug in tablets.

Tableted SODFs were prepared using a dry granulation technique followed by compressing and film coating. Dry granulation by roller compaction was performed to minimize SODF moisture exposure during granulation process. The general production process involved SODF lubrication using intragranular fillers followed by roller compaction and milling. Resulting SODF composition granules were mixed and lubricated with extragranular excipients to obtain final powder SODF mixtures, which were then compressed into tablets and coated with the Vivacoat PC-8T-181 or Opadry II White 85F18422 films.

API content in one tablet is given in Table 4.

**Table 4. Active ingredient and excipient contents in each tablet**

| Ingredient | SODF tablet | | | | | | |
|---|---|---|---|---|---|---|---|
| | SODF 1 | SODF 2 | SODF 3 | SODF 4 | SODF 5 | SODF 6 | SODF 7 |
| | Active ingredient content (mg) in SODF | | | | | | |
| **1a** | 20.7 | 20.7 | 20.7 | 20.7 | 20.7 | 20.7 | 20,7 |
| **2b** | 200 | | 200 | | | 200 | 200 |
| **2j** | | 250 | | 250 | 250 | | |
| **3** | 300 | 300 | | | | | |
| **4f** | | | 25 | | | | |
| **4h** | | | | 25 | | 25 | |
| **4m** | | | | | 25 | | 25 |
| Total*^{α}* (mg) | 520.7 | 570.7 | 245.7 | 295.7 | 295.7 | 295.7 | 295.7 |

| | Excipient content in a tablet (mg) | | | | | | |
|---|---|---|---|---|---|---|---|
| LM*^{b}* | 386.94 | | | | | | |
| MCC*^{c}* | 134.20 | | | | | | |
| CS*^{d}* | 67.08 | | | | | | |
| GS*^{e}* | 33.4 mg | | | | | | |
| Povidone K30 | 15.0 mg | | | | | | |
| MS*^{f}* | 10.68 | | | | | | |
| EW*^{g}* (mg) | 697.3 | | | | | | |
| TCW*^{h}* | 1168 | 1218 | 893 | 943 | 943 | 943 | 943 |
| FC*ⁱ* | 50,00 | | | | | | |
| TW*^{j}* (mg) | 1218 | 1268 | 943 | 993 | 993 | 993 | 993 |

| | | | | | | | |
|---|---|---|---|---|---|---|---|
| *^{α}*Total weight (mg) of active ingredients in a tablet, *^{b}*Lactose monohydrate (200 mesh). *^{c}*Microcrystalline cellulose 102. *^{d}*Croscarmellose sodium. *^{e}*Gelled starch. *^{f}*Magnesium stearate. *^{g}*Excipient weight. *^{h}*Tablet core weight. *ⁱ*Film coat Vivacoat PC-8T-181. *^{j}*Coated tablet weight. | | | | | | | |

### Industrial applicability

The invention could be used in medicine and veterinary.

## Claims

1. A combination drug in a solid dosage form for the treatment of viral infections comprising, as one of the three active ingredients, a therapeutically effective amount of elsulfavirine sodium of formula **1a** in a crystalline or polycrystalline form optionally in combination with auxiliary agents:

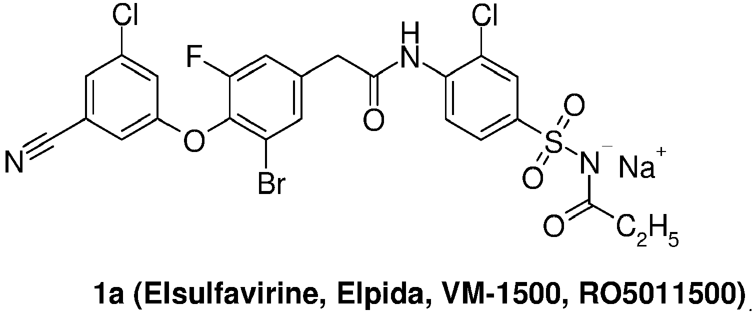

2. The combination drug according to claim 1 for the treatment of human immunodeficiency virus.

3. The combination drug according to claim 1 for the treatment of Hepatitis B Virus (HBV).

4. The combination drug according to claim 1 comprising in a crystalline or polycrystalline form, as the other two active ingredients, a therapeutically effective amount of a Nucleoside Reverse Transcriptase Inhibitor (NRTI) precursor of formulas **2a-2j** or a pharmaceutically acceptable salt thereof and a therapeutically effective amount of a Nucleotide Reverse Transcriptase Inhibitor (NtRTI) precursor of formula **3** or **4a-4m** or a pharmaceutically acceptable salt thereof:

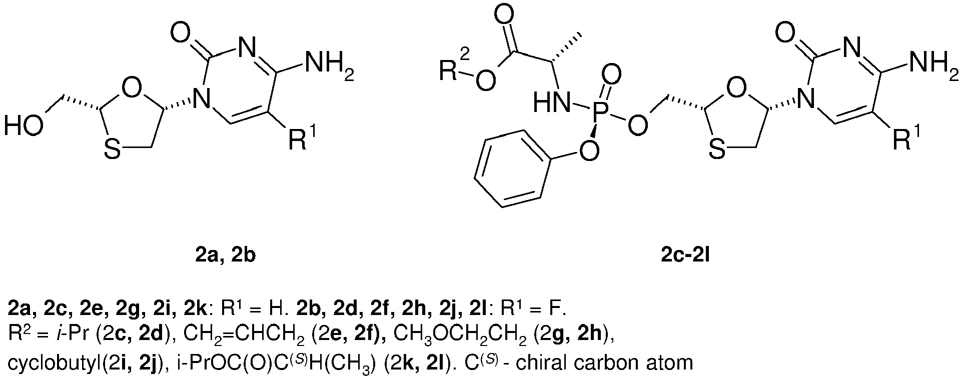

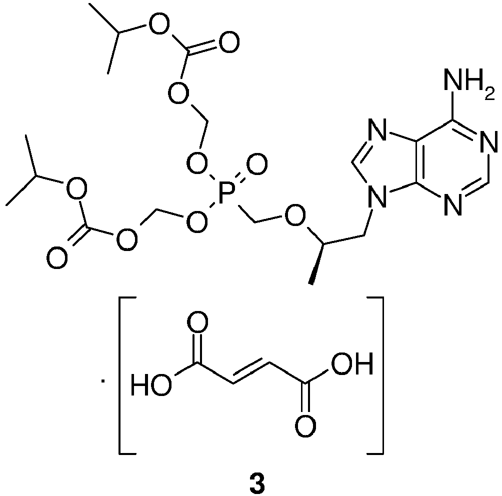

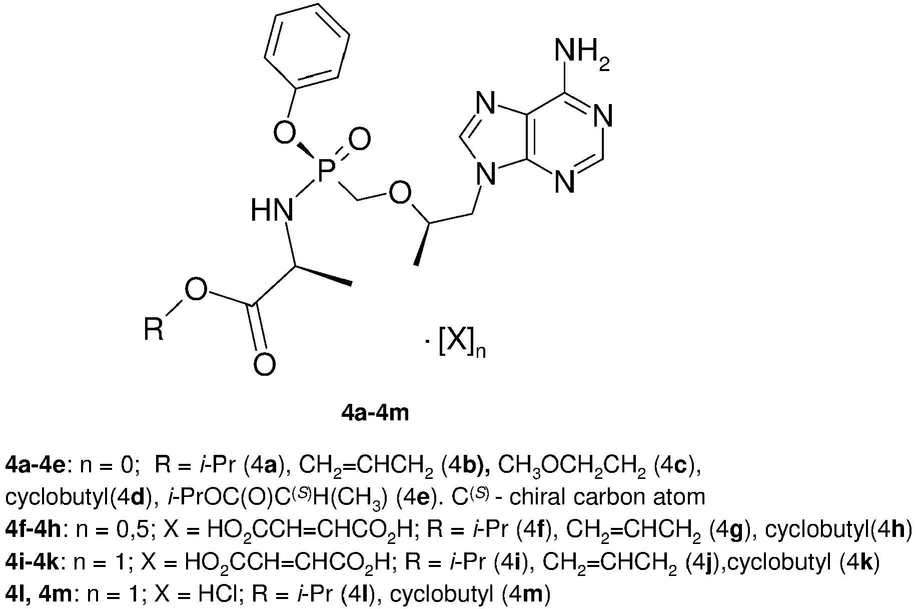

5. The combination drug according to any of claims 1 to 4 in the form of tablets, gelatin capsules, pills, powders, or chewing gums.

6. The combination drug according to any of claims 1 to 5 comprising, as active ingredients, elsulfavirine sodium of formula **1a,** an NRTI precursor of formulas **2a-2j** or a salt thereof, and tenofovir disoproxil fumarate of formula **3** in a mass ratio of **1a** : **2a-2j** or a salt thereof: **3 ≈** 1:10:15.

7. The combination drug according to any of claims 1 to 5 comprising, as active ingredients, 15-25 mg of elsulfavirine sodium of formula **1a**, 150-300 mg of an NRTI precursor of formulas **2a-2j** or a salt thereof, and 250-350 mg of tenofovir disoproxil fumarate of formula **3.**

8. The combination drug according to claims 6, 7 in tablets comprising, as excipients, lactose monohydrate 200, microcrystalline cellulose 102, croscarmellose sodium, pre-gelled starch, Povidone K30, magnesium stearate, and, as a film coating, Vivacoat PC-8T-181, with the mass ratio depending on their nature and mode of production.

9. The combination drug according to claim 8, wherein each tablet comprises 20.7 mg of elsulfavirine sodium of formula **1a,** 200-300 mg of an NRTI precursor of formulas **2a-2j,** 300 mg of tenofovir disoproxil fumarate of formula **3,** 386.9 mg of lactose monohydrate 200, 134.2 mg of monocrystalline cellulose 102, 67.1 mg of croscarmellose sodium, 33.4 mg of pre-gelled starch, 15.0 mg of Povidone K30, 10.7 mg of magnesium stearate, and, as a film coating, 50.0 mg of Vivacoat PC-8T-181.

10. The combination drug according to any of claims 1 to 5 comprising, as active ingredients, elsulfavirine sodium of formula **1a,** an NRTI precursor of formulas **2a-2j** or a salt thereof, and tenofovir of formulas **4a-4m** in a mass ratio of **1a** : **2a-2j** or a salt thereof: **4a-4m** ≈ 1:10:1,25.

11. The combination drug according to any of claims 1 to 5 comprising, as active ingredients, 15-25 mg of elsulfavirine sodium of formula **1a**, 150-300 mg of an NRTI precursor of formulas **2a-2j** or a salt thereof, and 10-35 mg of tenofovir of formulas **4a-4m.**

12. The combination drug according to any of claims 1 to 5 comprising, as active ingredients, 15-25 mg of elsulfavirine sodium of formula **1a**, 150-300 mg of an NRTI precursor of formulas **2a-2j** or a salt thereof, and 10-35 mg of tenofovir of formula **4h** or **4m.**

13. The combination drug according to any of claims 1 to 5, 11 and 12 comprising, as active ingredients, 15-25 mg of elsulfavirine sodium of formula **1a,** 150-300 mg of an NRTI precursor of formulas **2a-2j** or a salt thereof, 10-35 mg of tenofovir of formulas **4a-4m;** as excipients, lactose monohydrate 200, microcrystalline cellulose 102, croscarmellose sodium, pre-gelled starch, Povidone K30, magnesium stearate; and, as a film coating, Vivacoat PC-8T-181.

14. The combination drug according to claim 13 comprising 20.7 mg of elsulfavirine sodium of formula **1a,** 200-300 mg of an NRTI precursor of formulas **2a-2j,** 25 mg of tenofovir of formula **4f, 4h** or **4m,** 386.9 mg of lactose monohydrate 200, 134.2 of mg monocrystalline cellulose 102, 67.1 mg of croscarmellose sodium, 33.4 mg of pre-gelled starch, 15.0 mg of Povidone K30, 10.7 mg of magnesium stearate, and, as a film coating, 50.0 mg of Vivacoat PC-8T-181.

15. The combination drug according to claim 12 comprising, as active ingredients, 10-25 mg of elsulfavirine sodium of formula **1a,** 150-350 mg of an NRTI precursor of formulas **2a-2j,** 5-35 mg of tenofovir of formula **4f, 4h** or **4m,** 20-35 mg of croscarmellose sodium, 70-120 mg of microcystalline cellulose, and 1-7 mg of magnesium stearate.

16. The combination drug according to claim 12 comprising, as active ingredients, 20.7 mg of elsulfavirine sodium of formula **1a,** 200-300 mg of an NRTI precursor of formulas **2a-2j,** 25 mg of tenofovir of formula **4f, 4h** or **4m,** 28 mg of croscarmellose sodium, 105.56 mg of cellulose microcrystals, 5.25 mg of magnesium stearate and a film coating consisting of Vivacoat PC-8T-181.

17. A method for producing a combination drug formulated in tablets by mixing therapeutically effective amounts of elsulfavirine sodium of formula **1a,** an NRTI precursor of formulas **2a-2j** or a pharmaceutically acceptable salt thereof, and an NtRTI precursor of formula **3** or formulas **4a-4m** or a pharmaceutically acceptable salt thereof with auxiliary agents followed by compression.

18. A method of treatment or prevention of viral diseases by oral administration to the patient of the novel combination drug according to claim 1.

19. The method according to claim 18 for treating or preventing HIV.

20. The method according to claim 18 for treating or preventing HBV.
